# EUROPEAN PATENT SPECIFICATION

(11) **EP 0 714 385 B1**
(45) Date of publication and mention of the grant of the patent: **11.11.1998**
(21) Application number: 94926211.7
(22) Date of filing: 18.08.1994
(51) Int. Cl.: C07C 29/16, C07C 31/12, C07C 31/125, C07C 31/27, C07C 45/50, C07C 47/02, C07C 47/12, C07C 47/133, C07C 51/373, C07C 67/29

(54) **HYDROFORMYLATION PROCESS**
HYDROFORMYLIERUNGS VERFAHREN
PROCEDE D'HYDROFORMYLATION

(30) Priority: 19.08.1993 EP 93202447; 30.12.1993 EP 93203727; 10.03.1994 EP 94200627
(43) Date of publication of application: 05.06.1996
(73) Proprietor: SHELL INTERNATIONALE RESEARCH MAATSCHAPPIJ B.V., 2596 HR Den Haag (NL)
(72) Inventor: DRENT, Eit, NL-1031 CM Amsterdam (NL); PELLO, Dennis, Humphrey, Louis, NL-1031 CM Amsterdam (NL); SUYKERBUYK, Jacoba, Catherina, Lucia, Johanna, NL-1031 CM Amsterdam (NL); VAN GOGH, Johan, NL-2596 HR The Hague (NL)
(86) International application number: EP9402762
(87) International publication number: WO9505354

(56) References cited:
- EP-A- 0 220 767
- EP-A- 0 495 547
- EP-A- 0 529 698

## Description

The invention relates to a process for the hydroformylation of ethylenically unsaturated compounds by reaction thereof with carbon monoxide and hydrogen in the presence of a catalyst.

The hydroformylation of ethylenically unsaturated compounds to form aldehydes and/or alcohols, is of considerable industrial importance. The process has been in commercial operation for decades and over the years much development work has been done to optimize the reaction conditions, the catalyst system and the equipment. Although significant progress as regards higher yield and selectivity to the desired reaction products has been made, it is felt that in some aspects further improvement of the process is still needed.

Conventional modes of operation were initially based on the use of a cobalt carbonyl catalyst. The activity of this catalyst is relatively low and moreover, when using internal olefins as starting material, a mixture of hydroformylation products is formed containing substantial amounts of branched compounds. For many applications the presence of these branched compounds is undesirable. Moreover, in view of biological degradability, it is also considered advantageous to produce mixtures exhibiting high linear versus branching ratios.

The formation of branched products can be suppressed by using a cobalt-phosphine complex as catalyst. However at the relatively high reaction temperatures required for an adequate activity of this catalyst system, substantial amounts of saturated hydrocarbons (approximately 15%) are formed, in addition to the desired hydroformylation products.

It has been proposed to use a rhodium-based catalyst for hydroformylation reactions. A limitation of this catalyst consists in that with internal olefins as starting material, branched products are formed. In general, with a rhodium catalyst, the obtained hydroformylation product predominantly consists of aldehydes. For some uses, e.g., for applications in the detergent industry, alcohols are a preferred starting material. Attempts have therefore been made to enhance the formation of alcohols, rather than that of aldehydes, e.g., by increasing the hydrogen versus carbon monoxide ratio, but these modes of operation invariably result in the formation of substantial amounts of saturated hydrocarbons.

It would hence be attractive if reaction conditions could be selected and a catalyst could be found such that the production of these saturated compounds is minimized.

In EP-A-0,220,767 a hydroformylation process is described, whereby an ethylenically unsaturated compound having at least 5 carbon atoms per molecule is contacted with carbon monoxide and hydrogen in the presence of an aprotic solvent and a catalyst, based on palladium, platinum or a compound of one of these metals, an anion of a carboxylic acid with a pKa of less than 2 and a bidentate of the formula Q¹Q²MQMQ³Q⁴ wherein M represents phosphorus, arsenic or antimony, Q represents a divalent organic bridging group having at least three carbon atoms in the bridge and Q¹...Q⁴ are similar or dissimilar optionally substituted hydrocarbyl groups.

From the experimental results, revealed in the examples, it can be seen that under the selected reaction conditions, the conversion is about 65%, the amount of linear compounds in the product mixture is 67% and that, although predominantly aldehydes are obtained, also some formation of paraffins takes place.

In EP-A-0,495,547 a variety of carbonylation reactions are described including the hydroformylation of ethylenically unsaturated compounds. According to one of these examples (i.e., example 27) an alpha-olefin (1-octene) may be hydroformylated at 90 °C and 60 bar (1:1) hydrogen and carbon monoxide, using a catalyst, comprising palladium, 1,3-bis(diisopropylphosphino)propane and a sulphonic acid to form 94% nonanals and 5% nonanols, the conversion being about 67%. When the same experiment is carried out at 125 °C and 60 bar (2:1) hydrogen and carbon monoxide it results in the formation of 88% nonanols and 9% nonanals with an olefin conversion of 63%. The remainder therefore comprises 1, respectively 3% paraffins. Although suitable for the hydroformylation of alpha-olefins (initial rate of conversion in the order of about 300 mol/mol Pd.h), for the hydroformylation of internal olefins, this catalyst is found to be significantly less active (approximate rate of 25 to 30 mol/mol Pd.h).

Finally, in EP-A-0,529,698 a process is described for the hydrogenation of a carbonyl compound in the presence of a homogeneous catalytic system comprising a source of a Group VIII metal compound and a bidentate phosphine such as 1,3-bis(1,5-cyclooctylenephosphino)propane, which catalyst is also active as hydroformylation catalyst. Still the rate of conversion is only in the order of 45 mol/mol Pd.h or even less.

Surprisingly, it has now been found that by selecting a catalyst based on a metal of the platinum group, a bidentate ligand comprising at least one bivalent cyclic moiety, and a source of anions other than halide anions, improved hydroformylation results are achieved as regards, inter alia, conversion rate, linearity of product and suppression of paraffin make.

Moreover, the presence of a small amount of catalyst promoter comprising a source of halide anions has been found to have a significantly favourable effect in that the hydroformylation proceeds at high rate, even at moderate temperatures, with very little formation of saturated hydrocarbons.

The invention may be defined as relating to a process for the hydroformylation of ethylenically unsaturated compounds by reaction thereof with carbon monoxide and hydrogen in the presence of a catalyst system comprising:
a) a source of platinum group metal cations;
b) a source of anions, other than halide anions; and
c) a source of bidentate ligands of the formula I

   R¹R²M¹RM²R³R⁴ (I)
wherein M¹ and M² independently represent a phosphorus, arsenic or antimony atom, R represents a bivalent organic bridging group containing from 1 to 4 atoms in the bridge, R¹ and R² together represent a bivalent substituted or non-substituted cyclic group whereby the two free valencies are linked to M¹ and R³ and R⁴ independently represent a substituted or non-substituted hydrocarbyl group, or together represent a bivalent substituted or non-substituted cyclic group whereby the two free valencies are linked to M² and in the further presence of a catalyst promoter comprising a source of halide anions such that the molar ratio between halide and platinum group metal cations is at most 3:1.

In the present specification the metals of the platinum group are defined as the metals with the atomic numbers 28, 46 and 78, i.e. nickel, palladium and platinum. Of these, palladium and platinum are preferred.

Examples of suitable metal sources are platinum or palladium compounds such as salts of palladium and nitric acid, sulphuric acid or sulphonic acids, salts of platinum or palladium and carboxylic acids with up to 12 carbon atoms, palladium- or platinum complexes, e.g. with carbon monoxide or acetylacetonate, or palladium combined with a solid material such as an ion exchanger or carbon. Palladium(II) acetate and platinum(II) acetylacetonate are examples of preferred metal sources.

As anion source, other than halide anions, any compound generating these anions may be used. Suitably, acids, or salts thereof, are used as source of anions, for example any of the acids mentioned above, which may also participate in the salts of the metals of the platinum group.

In the catalyst systems of the invention, preferably strong acids are used as anion source, i.e. acids having a pKa value of less than 3, preferably less than 2, measured in aqueous solution at 18 °C. The anions derived from these acids are non-coordinating or weakly coordinating with the metals of the platinum group.

Typical examples of suitable anions are anions of phosphoric acid, sulphuric acid, sulphonic acids and halogenated carboxylic acids such as trifluoroacetic acid.

Sulphonic acids are in particular preferred, for example methanesulphonic acid, trifluoromethanesulphonic acid, tert-butanesulphonic acid, p-toluenesulphonic acid and 2,4,6-trimethylbenzenesulphonic acid.

Also, complex anions are suitable, such as the anions generated by a combination of a Lewis acid such as BF₃, AlCl₃, SnF₂, Sn(CF₃SO₃)₂, SnCl₂ or GeCl₂, with a protic acid, such as a sulphonic acid, e.g. CF₃SO₃H or CH₃SO₃H or a hydrohalogenic acid such as HF of HCl, or a combination of a Lewis acid with an alcohol. Examples of such complex anions are BF₄⁻, SnCl₃⁻, [SnCl₂.CF₃SO₃]⁻ and PF₆⁻.

In bidentate ligands of formula (I), i.e., component c of the catalyst system, M¹ and M² are preferably the same and, more preferably, are both phosphorus atoms, in which case the ligands are bisphosphines.

In the organic bridging group, represented by R, typically all bridging groups are carbon atoms. Preferably the bridging group contains two carbon atoms in the bridge. It has been observed that the reaction rate is usually considerably enhanced, if instead of a catalyst based on a three membered bridging group, for instance a trimethylene group, a catalyst is used based on a two membered bridging group, for example an ethylene group.

This is surprising, because in earlier hydroformylation processes, such as the ones disclosed in EP-A-0,220,767 and EP-A-0,495,547, bidentate ligands are used whereby the presence of a bridging group containing 3 or more carbon atoms is preferred, or, as in EP-A-0,220,767, even required.

The bivalent (substituted) cyclic group, represented by R¹ together with R², in general comprises at least 5 ring atoms and preferably contains from 6 to 9 ring atoms. More preferably the cyclic group contains 8 ring atoms. Substituents, if any, are usually alkyl groups having from 1 to 4 carbon atoms. As a rule, all ring atoms are carbon atoms, but bivalent cyclic groups containing one or two heteroatoms in the ring, such as oxygen- or nitrogen, atoms are not precluded. Examples of suitable bivalent cyclic groups are 1,4-cyclohexylene, 1,4-cycloheptylene, 1,3-cycloheptylene, 1,2-cyclooctylene, 1,3-cyclooctylene, 1,4-cyclooctylene, 1,5-cyclooctylene, 2-methyl-1,5-cyclooctylene, 2,6-dimethyl-1,4-cyclooctylene and 2,6-dimethyl-1,5-cyclooctylene groups.

Preferred bivalent cyclic groups are selected from 1,4-cyclooctylene, 1,5-cyclooctylene, and methyl (di)substituted derivatives thereof.

Mixtures of ligands comprising different bivalent cyclic groups may be used as well, e.g. mixtures of ligands with 1,4-cyclooctylene and ligands with 1,5-cyclooctylene groups.

In the ligands of formula (I), R³ and R⁴ may independently represent various non-cyclic or cyclic groups, optionally substituted with substituents such as alkoxy groups with 1 to 4 carbon atoms, halogen atoms or (C₁ to C₄ alkyl)amino groups.

Examples are alkyl groups such as ethyl, isopropyl, sec-butyl and tert-butyl groups, cycloalkyl groups such as cyclopentyl and cyclohexyl groups, aryl groups such as phenyl and tolyl groups and bivalent groups such as a hexamethylene group. However, preferably R³, together with R⁴ represents a bivalent cyclic group, in particular the same group as the group represented by R¹ together with R², in which case the two free valencies of the bivalent cyclic group are, of course, linked to M², instead of M¹. Thus, preferred bidentate ligands of formula (I) are 1,2-bis(1,4-cyclooctylenephosphino)ethane, 1,2-bis(1,5-cyclooctylenephosphino)ethane and mixtures thereof.

For the preparation of the bidentate ligands, reference is made to known techniques, for example the method disclosed in GB-A-1,127,965.

The quantity in which the catalyst system is used, is not critical and may vary within wide limits. Usually amounts in the range of 10⁻⁸ to 10⁻¹, preferably in the range of 10⁻⁷ to 10⁻² mole atom of platinum group metal per mole of ethylenically unsaturated compound are used. The amounts of the participants in the catalyst system are conveniently selected such that per mole atom of platinum group metal from 0.5 to 10, preferably from 1 to 6 moles of bidentate ligand are used, from 0.5 to 15, preferably from 1 to 8 moles of anion source or a complex anion source.

The process of the present invention consists in the presence of a catalyst promoter, comprising a source of halide anions, with the proviso that the molar ratio between halide anions and platinum group metal cations should be at most 3:1.

If larger amounts of halide anions are present, the activity of the catalyst system is adversely affected, presumably because of coordination occurring between palladium and halide moieties.

Preferably, the molar ratio between halide anions and platinum group metal cations is at most 2:1, more preferably less than 1:1, for instance from 0.02:1 to 1:1.

As source of halide anions any compound generating halide anions under the reaction conditions may be used.

Recommended are inorganic compounds such as hydrogen halides, e.g. HCl, HBr and HI and metal halides, e.g. NaCl, MgBr₂, ZnCl₂, ZnI₂, KBr, RbCl, CsCl, CsI, MgI₂ and CuCl.

Another category of recommended sources of halide anions consists of halogen containing organic compounds which are capable of providing halide anions to the reaction medium. Suitable are for example organic phosphonium halides, such as triarylalkyl phosphonium chloride and halogen containing aromatic compounds such as 5-halobenzoic acids, e.g. 5-chlorobenzoic acid, 2,5-dichlorobenzoic acid, 2,3,5-tri-iodobenzoic acid, 3,5-di-iodobenzoic acid, m-halophthalic acids and esters thereof.

Catalyst promoters comprising a source of chloride anions are in particular preferred.

The ethylenically unsaturated compound, used as starting material, is preferably an olefin having from 2 to 30 carbon atoms per molecule, or a mixture thereof. They may comprise one or more double bonds per molecule. Preferred are internal olefins having from 4 to 24 carbon atoms, or mixtures thereof. Such olefin mixtures are commercially readily available, for example the olefin mixtures, obtained as products of a process for the oligomerization of ethylene, followed by a double bond isomerization and disproportionation reaction. In the process of the invention, these internal olefins, usually mixtures of linear internal olefins with 6 to 20 carbon atoms per molecule, or closer boiling fractions of such mixtures, can be hydroformylated at high rates and an almost complete conversion. Examples are mixtures of linear internal C₆ to C₈ olefins, and of linear internal C₁₀ to C₁₄ olefins.

Substituted olefins may also be used, for example unsaturated carboxylic acids, esters of such acids, or unsaturated esters of carboxylic acids, e.g. allylacetate.

If desired, branched olefins such as propene trimer or isomeric butene dimers ("DIMERSOL" a trademark) may be used, but the hydroformylation product will then, of course, contain branched structures as well.

Also, olefinically unsaturated polymeric feedstock, such as atactic polyolefins like 'Shube's' (mixture of oligomers of C₁₆-olefins), "NAPVIS" and "HYVIS" (trademarks for low molecular weight polyisobutylene) and styrene-butadiene (block)copolymers may be converted into interesting alcohols (as intermediates to synthetic lubricants, functionalized additives, etc.).

Finally, alpha-olefins, such as 1-octene and propene, and diolefins, such as norbornadiene, dicyclopentadiene, 1,5-hexadiene and 1,7-octadiene may be used. The diolefins will of course yield (predominantly) a di-hydroformylated product, although also monohydroformylated may be formed.

Carbon monoxide and hydrogen may be supplied in equimolar or non-equimolar ratios, e.g. in a ratio within the range of 5:1 to 1:5, typically 3:1 to 1:3. Preferably they are supplied in a ratio within the range of 2:1 to 1:2.

The hydroformylation can be suitably carried out at moderate reaction conditions. Hence temperatures in the range of 50 to 200 °C are recommended, preferred temperatures being in the range of 70 to 160 °C. Reaction pressures in the range of 5 to 100 bar are preferred, lower or higher pressures may be selected, but are not considered particularly advantageous. Moreover, higher pressures require special equipment provisions.

In the process of the invention, the ethylenically unsaturated starting material and the formed hydroformylation product may act as reaction diluent. Hence, the use of a separate solvent is not necessary. Conveniently, however, the hydroformylation reaction may be carried out in the additional presence of a solvent. As such, saturated hydrocarbons, e.g. paraffins and isoalkanes are recommended and furthermore alcohols, preferably having from 4 to 10 carbon atoms per molecule, such as butanol, ethylhexanol-1, nonanol-1, or in general terms the alcohols formed as hydroformylation product; ethers such as 2,5,8-trioxanonane (diglyme), diethylether and anisole, and ketones, such as methylbutylketone.

In earlier hydroformylation processes, such as the process according to EP-A-0,495,547, the use of an alcohol as solvent was often considered undesirable, since the used hydroformylation catalysts were also catalytically active in the formation of esters in a reaction involving an olefin, carbon monoxide and the solvent alcohol. However, the catalyst systems of the present invention in view of their high selectivity towards the desired hydroformylation product, allow the use of alcohols as solvent.

It would moreover be advantageous, if the hydroformylation process could be carried out in a homogeneous reaction medium using a dissolved catalyst system of adequate activity, whereby nevertheless the catalyst, without significant loss or decomposition thereof, can be readily recovered and reused if so desired.

Accordingly, a preferred embodiment of the invention relates to a process for the hydroformylation of ethylenically unsaturated compounds having at least 4 carbon atoms by reaction thereof with carbon monoxide and hydrogen in a single-phase liquid medium, in the presence of the aforementioned catalyst system, followed by effecting the formation of a multiphase liquid reaction medium comprising one phase in which substantially all platinum group metal cations of the catalyst system is present and at least one further phase containing a major portion of the hydroformylated product.

The unsaturated compounds used in the process of this embodiment contain at least 4 carbon atoms per molecule. It has been established that with ethylenically unsaturated compounds having only 2 or 3 carbon atoms per molecule, the formation of a multi-phase liquid reaction medium, whereby the platinum group metal cations of the catalyst system is present in one phase and a major portion of the formed hydroformylated product in another phase, can not be easily effected. Preferably, ethylenically unsaturated compounds are used, having at least 6 carbon atoms per molecule.

In particular preferred are ethylenically unsaturated compounds having from 6 to 22 carbon atoms per molecule. A preferred starting material consists of internal linear olefins having from 12 to 16 carbon atoms per molecule.

The formation of the multiphase liquid reaction medium, following the hydroformylation reaction, can be effected in various ways.

For example, a selective solvent for the hydroformylated product may be added to the single-phase liquid reaction mixture, during or subsequent to the hydroformylation reaction, forming a second liquid phase in which a major portion of the hydroformylated product(s), and possibly part of any unconverted unsaturated starting material will be found.

Whereas in this embodiment two liquid phases are formed allowing an adequate separation between the catalyst system on the one hand and a major portion of the hydroformylation product on the other hand, it will be necessary to remove the hydroformylated product from the said second liquid phase, containing the added solvent.

Therefore, it is preferred to effect the formation of a multiphase liquid reaction medium by adding to the single-phase liquid reaction medium during or subsequent to the hydroformylation reaction, an inert solvent, capable of selectively dissolving substantially all platinum group metal of the catalyst system. This embodiment allows an adequate separation between the hydroformylated product and the catalyst system, at the same time simplifying the recovery of the hydroformylated product.

According to a still more preferred embodiment, the hydroformylation reaction is carried out in the presence of an inert solvent and, subsequent to the reaction, the multiphase liquid reaction medium is formed by cooling the reaction medium, obtained in the reaction.

In this manner it is possible to ensure, that substantially all platinum group metal of the catalyst system, i.e. at least 95% and in particular at least 97% of the platinum group metal, is present in the liquid phase, containing the inert solvent.

A major portion of the hydroformylated product, i.e. at least 50% and in particular at least 80% of the hydroformylated product is obtained in another liquid phase, from which it can be easily recovered by known techniques.

By selection of a suitable solvent the multiphase liquid medium is readily formed when the temperature of the reaction mixture is decreased to ambient temperatures. If desired, the reaction medium can be cooled to lower temperatures, but for large-scale operation this is not considered of special advantage, in view of the additional provisions to be made in the reactor section of the equipment.

Suitable inert solvents, which may be added instead of, or in addition to the solvents mentioned above, that are capable of selectively dissolving substantially all platinum group metal of the catalyst system, are usually characterized by the presence of an aprotic, polar group in the molecule.

Solvents containing strong polar groups are in particular preferred if the unsaturated starting material has a relatively low molecular weight, i.e., if ethylenically unsaturated compounds having from 5 to 7 carbon atoms are used.

For the hydroformylation of higher molecular weight unsaturated compounds, e.g. olefins having from 12 to 16 carbon atoms the use of less polar inert solvents will usually be satisfactory.

Solvents, comprising or substantially consisting of sulphones are preferred. Sulphones are in particular preferred, for example dialkylsulphones such as dimethylsulphone and diethylsulphone and cyclic sulphones, such as sulfolane (tetrahydrothiophene-2,2-dioxide), sulfolene, 2-methylsulfolane and 2-methyl-4-ethylsulfolane.

Sulfolane has proved to be a most effective solvent for the formation of a multiphase liquid reaction medium.

It has been found particularly beneficial to carry out the invention using sulfolane as solvent and a catalyst system based on a complex anion (for instance based on SnCl₂) and/or in the presence of a catalyst promoter, to counter the somewhat increased paraffin formation due to presence of the solvent.

Mixtures of solvents may also be used, for example a mixture of a sulphone with a protic solvent, such as an alcohol. In the hydroformylation of olefins, typically an alcohol is selected which is identical or similar to an alcohol as obtained in the hydroformylation reaction.

The amount of solvent to be used in the process of the invention may vary considerably. It is within the reach of those skilled in the art to establish in each case the degree of cooling and the optimal amount of solvent required for the formation of a multiphase liquid reaction medium. The experimental results provided hereinafter, are also indicative for the amount of solvent, preferably to be used.

The process of the invention is eminently suitable to be used for the preparation of alcohols from internal olefins at high rate, in particular by using a catalyst system as defined above, based on palladium as platinum group metal.

Furthermore the process is very useful for the preparation of aldehydes having a high linearity, in particular by using a catalyst system as defined above, based on platinum as platinum group metal.

The invention will be illustrated by the non-limiting examples, as described hereinafter. The abbreviations, used in the Tables have the following meanings:
BCPE = 1,2-bis(1,5-cyclooctylenephosphino)ethane
BDPE = 1,2-bis(2,6-dimethyl-1,5-cyclooctylenephosphino)ethane
TFSA = trifluoromethanesulphonic acid
t-BSA = tert-butanesulphonic acid
EH = 2-ethylhexan-1-ol

### Examples 1 - 14

The experiments were carried out in a 350 ml "HASTELLOY C" (HASTELLOY is a trademark) magnetically stirred autoclave.

The autoclave was charged with 30 ml of i-C₁₄=, 25 ml (or 45 ml) of EH, 6 ml of sulfolane, 0.25 mmol of palladium(II) acetate, 0.6 mmol of BCPE, 0.5 mmol of TFSA and a catalyst promoter.

After being flushed, the autoclave was pressurized with a 1:2 molar mixture of carbon monoxide and hydrogen up to a pressure of 60 bar and subsequently sealed. The temperature of the mixture was raised to the pre-set value.

After a reaction period of 3-10 hours, the reaction was discontinued and the reaction mixture cooled to ambient temperature.

A sample was taken from the contents of the reactor and analysed by Gas Liquid Chromatography. The i-C₁₄ = conversion was > 99%; further details and analytical results are compiled in Table I. The calculated conversion rate is expressed as moles of product per mole atom of platinum group metal and per hour (mol/mol.h). From this table it can be seen that the addition of a catalyst promoter is very beneficial in respect of the reduction of paraffin formation (cf. Examples 16 - 19 hereinafter).

### Example 15

An experiment was carried out, substantially as described with respect to Examples 1 - 14, with the difference that 45 ml of EH and, instead of BCPE, 0.6 mmol of BDPE was used.

The i-C₁₄ = conversion was > 99%. Further details and results are included in Table I.

### Comparative Example 16

A comparative experiment was carried out, substantially as described with respect to Examples 1 - 14, with the difference that no catalyst promoter was applied. The results are included in Table I to highlight the advantage of the copresence of a catalyst promoter.

### Comparative Example 17

A comparative experiment was carried out, substantially as described with respect to Examples 1 - 14, with the difference that instead of a catalyst promoter, 0.3 mmol of t-BSA was added. From the results, as given in Table I, it can be seen that the rate of the reaction is increased. Nonetheless, the production of paraffins is higher than in the experiments carried out in the presence of a catalyst promoter.

### Example 18

An experiment was carried out, substantially as described with respect to Examples 12 and 13, with the differences that the reaction temperature was 99 °C and that instead of 2,5-dichlorobenzoic acid, 5 mmol of 2,6-dichlorobenzoic acid was used as catalyst promoter. From the results, as given in Table I, it can be seen that by using an o,o-dihalo compound which will less easily generate halide anions, the reaction rate is increased, but that the paraffin production is not below 1%.

### Comparative Example 19

A comparative experiment was carried out, substantially as described with respect to Examples 12 and 13, with the difference that instead of 2,5-dichlorobenzoic acid, 5 mmol of 2,5-dihydroxybenzoic acid was used as catalyst promoter. From the results, as given in Table I, it can be seen that by using a promoter which is not capable of generating halide anions, the additional improvement achieved in the co-presence of a catalyst promoter as regards reaction rate and paraffin production is not achieved.

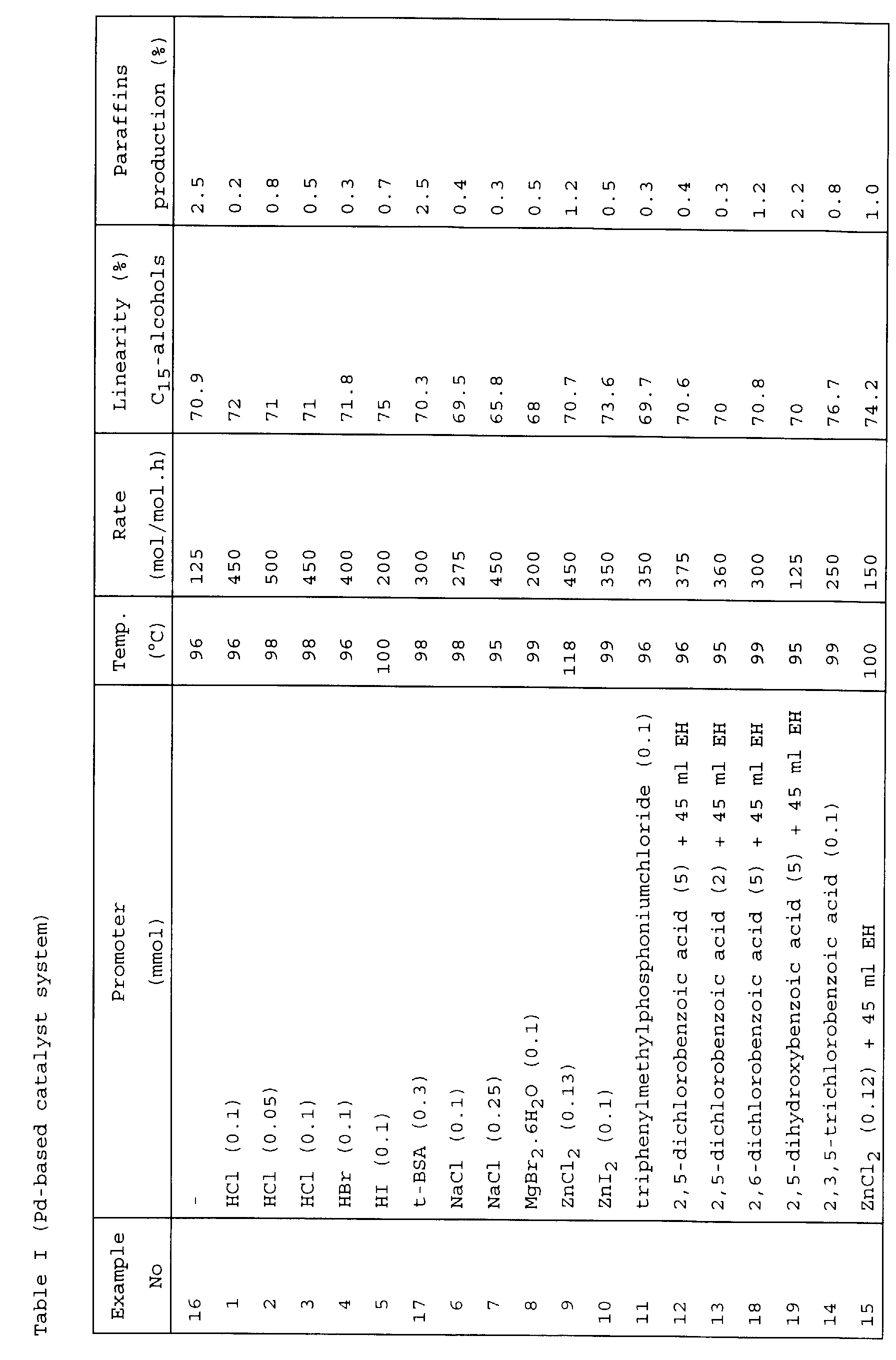

## Claims

1. A process for the hydroformylation of ethylenically unsaturated compounds by reaction thereof with carbon monoxide and hydrogen in the presence of a catalyst system comprising:
a) a source of platinum group metal cations;
b) a source of anions, other than halide anions; and
c) a source of bidentate ligands of the formula
R¹R²M¹RM²R³R⁴ (I)
wherein M¹ and M² independently represent a phosphorus, arsenic or antimony atom, R represents a bivalent organic bridging group containing from 1 to 4 atoms in the bridge, R¹ and R² together represent a bivalent substituted or non-substituted cyclic group whereby the two free valencies are linked to M¹ and R³ and R⁴ independently represent a substituted or non-substituted hydrocarbyl group, or together represent a bivalent substituted or non-substituted cyclic group whereby the two free valencies are linked to M² and in the further presence of a catalyst promoter comprising a source of halide anions such that the molar ratio between halide and platinum group metal cations is at most 3:1.

2. A process as claimed in claim 1, wherein the molar ratio between halide and platinum group metal cations is in the range from 0.02:1 to 1:1.

3. A process as claimed in any one of claims 1 or 2, wherein the catalyst system is based on a palladium or platinum compound.

4. A process as claimed in any one of claims 1 to 3, wherein the catalyst system is based on a source of bidentate ligands of formula (I), wherein M¹ and M² each represent a phosphorus atom.

5. A process as claimed in any one of claims 1 to 4, wherein in the bidentate ligand of formula (I) R represents an ethylene group.

6. A process as claimed in any one of claims 1 to 5, wherein in the bidentate ligand of formula (I) the bivalent cyclic group, represented by R¹ together with R², is a cycloalkylene group having from 6 to 9 ring atoms, preferably 8 ring atoms.

7. A process as claimed in any one of claims 1 to 6, wherein in the bidentate ligand of formula (I) R³ together with R⁴, has the same meaning as R¹ together with R², whereby the two free valencies are linked to M².

8. A process as claimed in any one of claims 1 to 7, for the hydroformylation of ethylenically unsaturated compounds having at least 4 carbon atoms by reaction thereof in a single phase liquid medium, followed by effecting the formation of a multiphase liquid reaction medium comprising one phase in which substantially all platinum group metal cations is present and at least one further phase containing a major portion of the hydroformylated product.

9. A process as claimed in claim 8, wherein the formation of the multiphase liquid reaction medium is effected with the aid of an inert solvent capable of selectively dissolving substantially all platinum group metal cations from a single phase liquid medium containing dissolved platinum group metal cations and hydroformylated product, such as sulfolane, an alkyl- or dialkylsulfolane, by cooling the reaction medium subsequent to the reaction.

## Patentansprüche

1. Verfahren zur Hydroformylierung von ethylenisch ungesättigten Verbindungen durch deren Umsetzung mit Kohlenmonoxid und Wasserstoff in Gegenwart eines Katalysatorsystems, das:
a) eine Quelle für Platingruppenmetallkationen;
b) eine von Halogenidanionen unterschiedliche Anionenquelle und
c) eine Quelle von Bidentatliganden mit der Formel
R¹R²M¹RM²R³R⁴ (I)
umfaßt, worin M¹ und M² unabhängig ein Phosphor-, Arsen- oder Antimonatom darstellen, R eine zweiwertige organische Brückengruppe mit einem Gehalt an 1 bis 4 Atomen in der Brücke bedeutet, R¹ und R² zusammengenommen eine zweiwertige substituierte oder unsubstituierte cyclische Gruppe bedeuten, wobei die beiden freien Valenzen an M¹ gebunden sind, und R³ und R⁴ unabhängig eine substituierte oder unsubstituierte Hydrocarbylgruppe darstellen oder zusammengenommen eine zweiwertige substituierte oder unsubstituierte cyclische Gruppe bedeuten, wobei die beiden freien Valenzen an M² gebunden sind, und in weiterer Gegenwart eines Katalysatorpromotors, der eine Quelle für Halogenidanionen umfaßt, derart, daß das Molverhältnis zwischen Halogenid und Platingruppenmetallkationen höchstens 3:1 beträgt.

2. Verfahren nach Anspruch 1, worin das Molverhältnis zwischen Halogenid und Platingruppenmetallkationen im Bereich von 0,02:1 bis 1:1 liegt.

3. Verfahren nach einem der Ansprüche 1 oder 2, worin das Katalysatorsystem auf einer Palladium- oder Platinverbindung aufgebaut ist.

4. Verfahren nach einem der Ansprüche 1 bis 3, worin das Katalysatorsystem auf einer Quelle von Bidentatliganden der Formel (I) beruht, worin M¹ und M² jeweils ein Phosphoratom darstellen.

5. Verfahren nach einem der Ansprüche 1 bis 4, worin in dem Bidentatliganden der Formel (I) R eine Ethylengruppe darstellt.

6. Verfahren nach einem der Ansprüche 1 bis 5, worin in dem Bidentatliganden der Formel (I) die zweiwertige cyclische Gruppe, dargestellt durch R¹ zusammengenommen mit R², eine Cycloalkylengruppe mit 6 bis 9 Ringatomen, vorzugsweise mit 8 Ringatomen ist.

7. Verfahren nach einem der Ansprüche 1 bis 6, worin in dem Bidentatliganden der Formel (I) R³ zusammengenommen mit F⁴ die gleiche Bedeutung wie R¹ zusammengenommen mit R² aufweisen, wobei die beiden freien Valenzen an M² gebunden sind.

8. Verfahren nach einem der Ansprüche 1 bis 7 zur Hydroformylierung von ethylenisch ungesättigten Verbindungen mit wenigstens 4 Kohlenstoffatomen durch deren Umsetzung in einem einphasigen flüssigen Medium und anschließende Ausbildung eines mehrphasigen flüssigen Reaktionsmediums, das eine Phase aufweist, worin im wesentlichen die Gesamtheit der Platingruppenmetallkationen vorliegt, und wenigstens eine weitere Phase aufweist, die einen Hauptteil des hydroformylierten Produktes enthält.

9. Verfahren nach Anspruch 8, worin die Ausbildung des mehrphasigen flüssigen Reaktionsmediums mit Hilfe eines inerten Lösungsmittels bewirkt wird, das zum selektiven Auflösen von im wesentlichen allen Platingruppenmetallkationen aus einem einphasigen flüssigen Medium, das gelöste Platingruppenmetallkationen und hydroformyliertes Produkt enthält, befähigt ist, wie Sulfolan, ein Alkyl- oder Dialkylsulfolan, durch Abkühlen des Reaktionsmediums nach der Umsetzung.

## Revendications

1. Procédé d'hydroformylation de composés éthyléniquement insaturés par leur réaction avec le monoxyde de carbone et l'hydrogène, en présence d'un système catalytique qui comprend :
a) une source de cations de métaux du groupe du platine,
b) une source d'anions autres que des anions halogénure et
c) une source de ligands bidentates de la formule
R¹R²M¹RM²R³R⁴ (I)
dans laquelle M¹ et M² représentent chacun indépendamment un atome de phosphore, d'arsenic ou d'antimoine, R représente un groupe de pontage organique bivalent comprenant de 1 à 4 atomes dans le pont, R¹ et R² représentent ensemble un groupe cyclique substitué ou non substitué bivalent où les deux valences libres sont liées à M¹ et R³ et R⁴ représentent chacun indépendamment un radical hydrocarbyle substitué ou non substitué, ou représentent ensemble un groupe cyclique substitué ou non substitué bivalent où les valences libres sont liées à M² et en la présence supplémentaire d'un promoteur de catalyseur comprenant une source d'anions halogénure, en sorte que le rapport molaire entre l'halogénure et les cations des métaux du groupe du platine atteigne au maximum 3:1.

2. Procédé suivant la revendication 1, caractérisé en ce que le rapport molaire entre l'halogénure et les cations de métaux du groupe du platine varie de 0,02:1 à 1:1.

3. Procédé suivant l'une quelconque des revendications 1 et 2, caractérisé en ce que le système catalytique est basé sur un composé du palladium ou du platine.

4. Procédé suivant l'une quelconque des revendications 1 à 3, caractérisé en ce que le système catalytique est basé sur une source de ligands bidentates de la formule (I), dans laquelle M¹ et M² représentent chacun un atome de phosphore.

5. Procédé suivant l'une quelconque des revendications 1 à 4, caractérisé en ce que, dans le ligand bidentate de la formule (I), R représente un groupe éthylène.

6. Procédé suivant l'une quelconque des revendications 1 à 5, caractérisé en ce que, dans le ligand bidentate de la formule (I), le groupe cyclique bivalent, représenté par R¹ ensemble avec R², est un groupe cycloalkylène possédant de 6 à 9 atomes cycliques, de préférence, 8 atomes cycliques.

7. Procédé suivant l'une quelconque des revendications 1 à 6, caractérisé en ce que, dans le ligand bidentate de la formule (I), R³ ensemble avec R⁴ possède la même signification que R¹ ensemble avec R², où les deux valences libres sont liées à M².

8. Procédé suivant l'une quelconque des revendications 1 à 7, pour l'hydroformylation de composés éthyléniquement insaturés possédant au moins 4 atomes de carbone par leur réaction dans un milieu liquide à phase unique, suivie de la formation d'un milieu de réaction liquide multiphase, comprenant une phase dans laquelle sensiblement tous les cations de métaux du groupe du platine sont présents et au moins une phase supplémentaire contenant une partie majeure du produit hydro-formylé.

9. Procédé suivant la revendication 8, caractérisé en ce que l'on entreprend la formation du milieu de réaction liquide multiphase à l'aide d'un solvant inerte capable de sélectivement dissoudre sensiblement tous les cations de métaux du groupe du platine, à partir d'un milieu liquide à phase unique contenant des cations de métaux du groupe du platine dissous et du produit hydroformylé, comme du sulfolane, un alkyl- ou dialkylsulfolane, par le refroidissement du milieu de réaction après la réaction.
